# EUROPÄISCHE PATENTSCHRIFT

(11) **EP 3 292 859 B1**
(45) Veröffentlichungstag und Bekanntmachung des Hinweises auf die Patenterteilung: **23.02.2022**
(21) Anmeldenummer: 17187403.5
(22) Anmeldetag: 23.08.2017
(51) Int. Cl.: A61K 9/06, A61K 31/07, A61K 31/122, A61K 31/355, A61K 31/592, A61P 3/02

(54) **HOCHDOSIERTE, KAUBARE VITAMINFORMULIERUNGEN**
HIGH DOSE, CHEWABLE VITAMIN FORMULATIONS
PRÉPARATIONS VITAMINIQUES À MÂCHER FORTEMENT DOSÉES

(30) Priorität: 23.08.2016 EP 16185349
(43) Veröffentlichungstag der Anmeldung: 14.03.2018
(73) Patentinhaber: IpSiCo UG, 68199 Mannheim (DE)
(72) Erfinder: BREITENBACH, Sarah Carolina, 68199 Mannheim (DE)
(74) Vertreter: Reitstötter Kinzebach

(56) Entgegenhaltungen:
- US-A1- 2004 247 746
- Amapharm: "YaYa Bären Multivitamin", , 23. Juni 2016 (2016-06-23), XP002777614, Gefunden im Internet: URL:https://api.gebrauchs.info/9195bffa28f d1012efbd828ed427cf2ai [gefunden am 2018-01-25]
- Volker Bühler: "Vademecum for vitamin formulations", 2001, Wissenschaftliche Verlagsslschaft mbH, Stuttgart, XP002777615, ISBN: 3-8047-1834-5 Seite 114, * Seite 114, Spalte 2 *

## Beschreibung

Die vorliegende Erfindung betrifft eine hochdosierte Vitaminformulierung in Form einer kaubaren und/oder lutschbaren Masse auf Basis eines Hydrokolloid-Gels. Die Erfindung betrifft ferner die Verwendung dieser Vitaminformulierung bei der Behandlung von Vitaminmangelzuständen insbesondere aufgrund von Vitaminmaldigestion bei Kindern oder alten Menschen.

Vitaminmaldigestion ist ein Symptom bestimmter Erkrankungen wie z.B. Mukoviszidose. Mukoviszidose, auch zystische Fibrose genannt, ist eine autosomal-rezessiv vererbte Stoffwechselerkrankung, deren Ursache in einer durch Mutation bedingten Fehlfunktion von Chloridkanälen bestimmter Körperzellen liegt, die zu einer Veränderung der Sekretzusammensetzung aller exokriner Drüsen des Patienten führt. Die betroffenen Zellen des Patienten sind nicht in der Lage, mittels Osmose Wasser in das umliegende Gewebe zu ziehen, wodurch der Wassergehalt des Bronchialsekrets sowie der Sekrete der Bauchspeicheldrüse, der Leber (Galle), der inneren Geschlechtsorgane und der akzessorischen Geschlechtsdrüsen sowie des Dünndarms zu niedrig ist. Dadurch werden die Sekrete zähflüssig und es kann in den betroffenen Organen zu Funktionsstörungen unterschiedlicher Art kommen. Mukoviszidose ist daher eine Multisystemerkrankung. Typische Symptome der Mukoviszidose im Bereich der Verdauung sind Maldigestion, Mangelernährung, Verdauungsstörungen, chronische Durchfälle sowie Untergewicht. Dazu gehört auch eine verringerte Aufnahmefähigkeit für insbesondere fettlösliche Vitamine und Provitamine, die zu chronischen Mangelzuständen führen kann. Bei der Behandlung von Mukoviszidosepatienten wird versucht dies auszugleichen, indem dem Patienten größere Mengen an diesen (Pro-)Vitaminen verabreicht werden.

Insbesondere bei der Behandlung von chronischen Mangelzuständen sind hochdosierte Vitaminformulierungen wünschenswert, um die Anzahl der vom Patienten einzunehmenden Tabletten oder Kapseln zu verringern. Des Weiteren ist es insbesondere für Kinder oder alte Menschen wünschenswert, dass die Vitaminformulierungen nicht zwingend durch Schlucken einer ganzen Tablette oder Kapsel eingenommen werden müssen, sondern gekaut und/oder gelutscht werden können. Jedoch führen hohe Konzentrationen an fettlöslichen Vitaminen oftmals zu einem unangenehmen Mundgefühl bzw. einem schlechten Geschmack. Beispielsweise führen hohe Konzentrationen an β-Carotin (dem Hauptvertreter der A-Provitamine) oft zu einem Geschmack, der als "fad", "taub" oder "alt" beschrieben wird. Während Vitamin-D-Präparate häufig als bitter oder metallisch schmeckend empfunden werden. Ferner ist bekannt, dass Formulierungen fettlöslicher Vitamine, insbesondere solche mit Vitamin D oder (Pro-)Vitamin A, in der Regel einen schlechten Geschmack haben, der v.a. im Fall von ölbasierten Formulierungen leicht zum Erbrechen führen kann. Dies stellt, insbesondere bei Kindern, ein Problem für die Akzeptanz kaubarer und/oder lutschbarer Formulierungen dar, die eine hohe Konzentration an derartigen Vitaminen und/oder Provitaminen enthalten.

Eine Kaumasse, die eines oder mehrere der Vitamine A, D, E und K enthält, ist in der Gebrauchsmusterschrift DE 202 004 010 021 beschrieben. Diese Kaumasse wird hergestellt, indem zunächst die Vitamine mit einem Emulgator, wie Polysorbat 80 oder Polysorbat 20, gemischt und bis zur Bildung eines klaren, homogenen Solubilisats gerührt werden. Das Solubilisat wird dann in ein Verdickungsmittel eingearbeitet. Dadurch enthält die resultierende Kaumasse eine recht hohe Emulgatorkonzentration. Eine lutsch- oder kaubare Gelatinemasse sind YaYa-Bären (Internet; URL:https:// api.gebrauchs.info/9195bffa28fd1012efbd828ed427cf2ai). Ein YaYa-Bär wiegt 1800 mg und enthält 6 mg Vitamin E, 200 µg Vitamin A und 2.5 µg Vitamin D3.

Auch EP-A-1338271 beschreibt die Herstellung eines Vitaminsolubilisats, bei dem ein fettlösliches Vitamin (a-Tocopherol, Retinol, β-Carotin) mit Polysorbat 20 oder Polysorbat 80 gemischt und bis zur Bildung eines klaren Solubilisats gerührt wird.

Die Verwendung von Emulgatoren, wie z.B. Polysorbaten, bei der Herstellung von oralen Formulierungen hat generell den physiologischen Nachteil, dass derartige Verbindungen insbesondere bei wiederholter Anwendung das Verdauungssystem negativ beeinflussen und zu Durchfällen führen können. Derartige Nebenwirkungen sind insbesondere nach operativen Eingriffen am Gastrointestinaltrakt oder bei Patienten, bei denen das Verdauungssystem deutlich beeinträchtigt ist, z.B. bedingt durch eine Krankheit wie Mukosviszidose, sehr nachteilig. Auch die Verabreichung ölhaltiger Zubereitungen solubilisierter fettlöslicher Vitamine, wie sie etwa in Form ölgefüllter Weichgelatinekapseln üblich sind, kann sich auf Patienten mit einem beeinträchtigten Verdauungssystem sehr nachteilig auswirken und ist daher zu vermeiden.

Es war daher wünschenswert, eine hochdosierte Vitaminformulierung bereitzustellen, die einfach durch Kauen und/oder Lutschen eingenommen werden kann, die das Verdauungssystem der Patienten schont, und trotz hohem Anteil an fettlöslichen (Pro-)Vitaminen, wie z.B. Vitamin A, β-Carotin, Vitamin D und Vitamin E, einen guten Geschmack aufweist.

Diese Aufgabe löst die vorliegende Erfindung durch Bereitstellung einer kaubaren und/oder lutschbaren Masse auf Basis eines Hydrokolloid-Gels, enthaltend
- Retinylacetat und β-Carotin
- mindestens ein Vitamin oder Provitamin ausgewählt unter den Vitaminen E, D und K sowie Provitaminen dieser Vitamine,
- Zink
   wobei
- die Gesamtmenge an den Vitaminen D, A, E und K und den Provitaminen dieser Vitamine, bezogen auf die Gesamtmasse des Hydrokolloid-Gels, mindestens 0,5 Masse-%, z.B. 0,5-0,6 Masse-% beträgt,
- die Gesamtmenge an Vitamin A und Provitamin A, bezogen auf die Gesamtmasse des Hydrokolloid-Gels, mindestens 0,050 Masse-%, z.B. 0,050-0,600 Masse-%, beträgt,
- die Masse 0,00-0,6 Masse-%, bezogen auf die Gesamtmasse des Hydrokolloid-Gels, Vitamin C enthält,
- der Wassergehalt des Hydrokolloid-Gels 1-35 Masse-%, vorzugsweise 5-30 Masse-%, z.B. 10-20 Masse-%, beträgt, und
- die Vitamine und Provitamine im Hydrokolloid-Gel in nicht Emulgator-solubilisierter Form vorliegen, wobei das Hydrokolloid-Gel vorzugsweise im Wesentlichen frei von Emulgatoren ist.

Bevorzugt enthält die erfindungsgemäße Masse Vitamin D oder Provitamin D.

Die Gesamtmenge an Vitamin D oder Provitamin D, bezogen auf die Gesamtmasse des Hydrokolloid-Gels, beträgt vorzugsweise mindestens 0,0003 Masse-%, insbesondere mindestens 0,001 Masse-%, vorzugsweise mindestens 0,005 Masse-%, z.B. 0,0003-0,060 Masse-%, 0,001-0,040 Masse-% oder 0,005-0,025 Masse-%.

Im Allgemeinen liegen mindestens 50 Masse-%, insbesondere mindestens 60 Masse-%, z.B. mindestens 70 Masse-%, mindestens 80 Masse-%, vorzugsweise mindestens 90 Masse-% und besonders bevorzugt 100 Masse-%, der Gesamtmenge an den Vitaminen A, D, E und K, und Provitaminen davon, in nicht-kristalliner (amorpher) Form vor.

In der erfindungsgemäßen Masse liegen die Vitamine und Provitamine in nicht Emulgator-solubilisierter Form vor, d.h. sie sind nicht durch Vorbehandlung mit Emulgatoren in eine wasserlösliche Form gebracht. Die Vitamine und Provitamine können zur Herstellung der erfindungsgemäßen Masse in Substanz, z.B. in kristalliner oder öliger Form, oder in vordispergierter bzw. redispergierbarer Pulverform eingesetzt werden. Obgleich die Vitamine und Provitamine in nicht Emulgator-solubilisierter Form vorliegen, wurde gefunden, dass die in der erfindungsgemäßen Masse enthaltenen Vitamine und Provitamine gut resorbiert werden können. Vermutlich bewirkt die Hydrokolloidgel-Matrix neben einer Geschmacksmaskierung eine Stabilisierung eines amorph dispergierten Zustands und ermöglicht eine gute Solubilisierung und Resorption.

Die erfindungsgemäße kaubare und/oder lutschbare Masse zeichnet sich durch einen sehr hohen Gehalt an fettlöslichen Vitaminen, wie Vitamin A, nämlich Retinylacetat, Vitamin D, E und/oder K, bzw. Provitaminen, wie Provitamin A, nämlich β-Carotin aus, die zudem überwiegend in gelöster, amorpher oder teilkristalliner Form vorliegen und damit eine signifikante Oberfläche darstellen, die beim Verzehr mit der Zunge als Geschmacksorgan in Kontakt treten kann. Trotzdem besitzt die Masse, im Gegensatz zu bekannten Vitaminformulierungen, überraschenderweise einen guten Geschmack. Ferner war überraschend, dass die erfindungsgemäße Masse, ohne dadurch einen unangenehmen Geschmack zu erhalten, einen signifikanten Anteil an Zinksulfat aufweisen kann, dessen Geschmack als "scharf metallisch salzig" beschrieben wird. Dies alles stellt einen enormen Vorteil hinsichtlich der Akzeptanz der erfindungsgemäßen Masse bei den Patienten, und insbesondere bei Kindern, dar bzw. ermöglicht eine Akzeptanz gar erst.

Die erfindungsgemäße kaubare und/oder lutschbare Masse enthält eine hohe Menge an fettlöslichen Vitaminen und/oder Provitaminen, wie Vitamin A, nämlich Retinylacetat, Provitamin A, nämlich β-Carotin, Vitamin D, Vitamin E und/oder Vitamin K. Dadurch können mit einer für den Patienten akzeptablen Menge (z.B. 20 g der Masse) 100% oder mehr der empfohlenen Tagesdosis an diesen Vitaminen verabreicht werden (siehe z.B. EU-RDA ("Recommended Daily Allowance") nach Richtlinie 2008/100/EG).

Die vorgenannten (Pro-)Vitamine sind nichtpolare Moleküle, die sehr gut in Lipiden löslich sind und in ähnlicher Weise wie Cholesterin in die Zellen der Darmschleimhaut in Chylomikronen eingebaut werden können.

Der Begriff "Vitamin D" bezeichnet mehrere chemische Verbindungen, wie etwa Vitamin D₃ (Cholecalciferol, Calciol), Vitamin D₂ (Calciferol, Ergocalciferol) und Vitamin D₄ (22,23-Dihydroergocalciferol, gesättigtes D₂). Als "Provitamin D" wird insbesondere Provitamin D₂ (Ergosterin) und Provitamin D₃ (7-Dehydrocholesterin) bezeichnet. In der erfindungsgemäßen Masse wird als Vitamin D bzw. Provitamin D bevorzugt Vitamin D₃ verwendet.

Der Begriff "Vitamin A" bezeichnet mehrere chemische Verbindungen, die teilweise direkt aus der Nahrung aufgenommen oder auch aus Provitamin A gebildet werden können. Beim Menschen zählt man z.B. Retinal, Retinol, 3-Dehydroretinol, Retinsäuren (z.B. All-trans-Retinsäure und 13-Cis-Retinsäure) und Retinale (z.B. All-trans-Retinal und 11-Cis-Retinal) als Vitamin A. Als "Provitamin A" werden Vitamin A-Derivate, wie z.B. Retinylpalmitat, Retinylacetat und Retinylpropionat, sowie insbesondere Carotine, wie z.B. β-Carotin, α-Carotin, γ-Carotin und β-Cryptoxanthin, bezeichnet. In der erfindungsgemäßen Masse wird als Vitamin A bzw. Provitamin A Retinylacetat und β-Carotin verwendet.

Der Begriff "Vitamin E" bezeichnet mehrere chemische Verbindungen, wie etwa Tocopherole (z.B. α-Tocopherol, β-Tocopherol, γ-Tocopherol, δ-Tocopherol, 5,7-Dimethyltocol, und 7-Methyltocol), Tocomonoenole (z.B. α-Tocomonoenol, β-Tocomonoenol, γ-Tocomonoenol und δ-Tocomonoenol), Tocotrienole (z.B. α-Tocotrienol, β-Tocotrienol, γ-Tocotrienol und δ-Tocotrienol) und marine Tocopherole ("marine-derived tocopherol" = MDT, z.B. α-MDT, β-MDT, γ-MDT, δ-MDT). Als "Provitamin E" werden Vitamin E-Derivate, wie etwa Tocopherylacetate (z.B. D,L-α-Tocopherylacetat und D-α-Tocopherylacetat) und Tocopherylsuccinate (z.B. D,L-α-Tocopherylsuccinat und D-α-Tocopherylsuccinat), bezeichnet. In der erfindungsgemäßen Masse wird als Vitamin E bzw. Provitamin E bevorzugt D,L-α-Tocopherylacetat oder D-α-Tocopherylacetat verwendet.

Der Begriff "Vitamin K" oder "Koagulationsvitamin" bezeichnet mehrere chemische Verbindungen, die sich von 2-Methyl-1,4-naphtochinon ableiten, wie etwa Vitamin K₁ (Phyllochinon), Vitamin K₂ (Menachinon), Vitamin K₃ (Menadion) und Vitamin K₄ (Menadiol). Als "Provitamin K" können insbesondere Vitamin K-Addukte, wie z.B. Hydrogensulfit-Vitamin-K₃-Addukt, bezeichnet werden. In der erfindungsgemäßen Masse wird als Vitamin K bzw. Provitamin K bevorzugt Vitamin K₁ verwendet.

Die erfindungsgemäße Masse enthält Retinylacetat und β-Carotin sowie mindestens ein Vitamin oder Provitamin ausgewählt unter Vitamin E, D und K sowie Provitamin E, D und K. Gemäß einer bevorzugten Ausführungsform enthält die erfindungsgemäße Masse Retinylacetat und β-Carotin, mindestens ein Vitamin E oder Provitamin E, mindestens ein Vitamin D oder Provitamin D sowie mindestens ein Vitamin K oder Provitamin K.

In der erfindungsgemäßen Masse liegen im Allgemeinen mindestens 50 Masse-% der Gesamtmenge an den Vitaminen und Provitaminen A, E, D und K in nicht-kristalliner (amorpher) Form vor. Untersuchungen zur Ermittlung der Kristallinität können mittels Weitwinkelröntgenbeugung (WAXS) oder durch differentialkalorimetrische Untersuchungen (DSC) durchgeführt werden. Besonders geeignet ist auch Lichtmikroskopie.

Die Vitamine und/oder Provitamine A, E, D und/oder K sind im Hydrokolloid-Gel typischerweise homogen verteilt (dispergiert). Bevorzugt liegen die mindestens 50 Masse-% an Vitaminen und Provitaminen A, E, D und K in Form einer festen Lösung (d.h. molekulardispers) im Hydrokolloid-Gel vor. Der Begriff "feste Lösungen" ist dem Fachmann geläufig, siehe dazu Chiou und Riegelman, J. Pharm. Sci. 60, 1281-1302 (1971). Es war überraschend, dass eine derartige Verteilung der fettlöslichen (Pro-)Vitamine auch in Gegenwart von Wasser möglich ist.

Das in der erfindungsgemäßen Masse enthaltene Hydrokolloid ist ausgewählt unter wasserlöslichen und wasserquellbaren gelbildenden Polysacchariden und Proteinen. Dazu gehören insbesondere:
- Pektine (Galacturonsäure-reiche Polysaccharide) wie z.B. Homoglacturonane, substituierte Galacturonane (mit terminalen D-Xylose- oder D-Apioseresten), Rhamnogalacturonan-I-Pektine, Rhamnogalacturonan-II-Pektine, und insbesondere Pektine mit einem zahlenmittleren Molekulargewicht im Bereich von 60-130.000;
- Gummiarabikum;
- Galactomannane wie z.B. Guarkernmehl, Carubin (aus Johannisbrotbaum) Konjak (aus der Teufelszunge);
- Agar;
- Carrageen wie z.B. κ-, 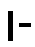 und λ-Carrageen;
- Alginate;
- Alginsäuren;
- Gelatine wie z.B. säurebehandelte Gelatine (wenig quervernetztes Kollagen) oder basenbehandelte Gelatine (stark quervernetztes Kollagen), die beispielsweise aus Rindern, Schweinen oder Fischen gewonnen werden kann, und insbesondere Gelatine mit einem Bloomwert (Gallertfestigkeit) von 30-300 und/oder einem zahlenmittleren Molekulargewicht von 15.000-25.000;
- Xanthan, insbesondere als Gemisch mit Johannisbrotkernmehl;
- Traganth; und
- Gemische dieser Hydrokolloide.

Dabei sind Gelatine und Pektine bevorzugt.

Die Gesamtmasse des Hydrokolloid-Gels bezieht sich auf die Gesamtmasse des/der gelbildenden Hydrokolloid(e) und des darin eingeschlossenen Lösungsmittels (Wasser).

Neben Vitaminen bzw. Provitaminen A, E, D und K kann die erfindungsgemäße kaubare und/oder lutschbare Masse weitere Substanzen mit biologischer Wirkung enthalten, wie z.B. weitere Carotinoide (Lycopin, Lutein, Zeaxanthin), Coenzyme (z.B. Coenzym Q10), α-Liponsäure, L-Carnitin, wasserlösliche, ggf. solublisierte Vitamine (z.B. B-Vitamine wie Vitamin B6 und Vitamin B12, sowie Folsäure), Fettsäuren, Aminosäuren (z.B. L-Tryptophan), Cofaktoren (z.B. Zink), Phytosterine, (natürlich vorkommende) Polyphenole, Flavonoide, Flavone, Antioxidantien (z.B. Vitamin C) und Aminosulfonsäuren (z.B. Taurin). In der erfindungsgemäßen Masse wird als Antioxidans bevorzugt Vitamin C und als Cofaktor Zink verwendet.

Vitamin C wird vorzugsweise nur in einer niedrigen Dosis als Antioxidans für die fettlöslichen Vitamine und Provitamine und nicht selbst als Vitaminkomponente zugesetzt. Beispielsweise liegt der Anteil an Vitamin C in der erfindungsgemäßen Masse bei 0,01-0,5 Masse-%, vorzugsweise 0,02-0,4 Masse-%, z.B. 0,03-0,4 Masse-%, bezogen auf die Gesamtmasse des Hydrokolloid-Gels.

Die Anwesenheit von Zink in der erfindungsgemäßen Masse ist vorteilhaft für den Vitamin-A-Stoffwechsel. Dieser wird im Wesentlichen durch Retinol-Bindeproteine gesteuert, die für die Nutzbarmachung von Vitamin A für den Körper benötigt werden. Ein Mangel an diesen Proteinen kann daher zu ähnlichen Symptomen führen wie ein Vitamin-A-Mangel selbst. Kann überschüssiges Retinol nicht durch Retinol-Bindeproteine gebunden werden, so treten Vergiftungserscheinungen auf. Retinol-Bindeproteine spielen daher auch bei einer Hypervitaminose A eine entscheidende Rolle. Da Retinol-Bindeproteine einen sogenannten Zinkfinger besitzen, ist das Spurenelement Zink wichtig für den gesamten Vitamin-A-Haushalt - sowohl bei Unter- als auch Überversorgung - und daher in der erfindungsgemäßen Masse enthalten. Geeignete Mengen an Zink können im Bereich von 0,001-0,1 Masse-%, insbesondere von 0,001-0,08 Masse-%, vorzugsweise 0,005-0,07 Masse-%, z.B. 0,01-0,06 Masse-%, bezogen auf die Gesamtmasse des Hydrokolloid-Gels, liegen. Das Zink liegt dabei zweckmäßigerweise in bioverfügbarer Form, z.B. als Zinksulfat, vor.

Die erfindungsgemäße Masse kann ein oder mehrere Süßungsmittel enthalten, wie etwa Zucker (z.B. Glukose, Fructose, Laktose, Saccharose, Invertzucker, Glukosesirup), Zuckeraustauschstoffe (z.B. Sorbitol, Mannitol, Xylitol, Maltitol) oder künstliche Süßstoffe (z.B. Saccharin, Natriumcyclamat, Acesulfam-K, Aspartam).

Weitere galenische Hilfsmittel wie Bindemittel, Sprengmittel, Geschmacksstoffe, Farbstoffe, Netzmittel, der pH-Wert beeinflussende Zusätze (siehe Sucker et al., Pharmazeutische Technologie, Thieme-Verlag, Stuttgart 1978) können ebenfalls in der Masse enthalten sein.

Zweckmäßigerweise ist die Zusammensetzung der erfindungsgemäßen Masse auf den Bedarf der Patienten zugeschnitten. Das heißt, sie enthält eine ausreichende Konzentration an den vom Patienten benötigten fettlöslichen (Pro-)Vitaminen und einen relativ geringen Anteil anderer Substanzen, die das Verdauungssystem der Patienten evtl. (weiter) beeinträchtigen könnten.

So besteht in einer Ausführungsform der Erfindung das in der Masse enthaltene Hydrokolloid-Gel zu 95-100 Masse-%, insbesondere zu 96-100 Masse-% oder zu 97-100 Masse-% und bevorzugt zu 98-100 Masse-%, aus den fettlöslichen Vitaminen bzw. Provitaminen A, E, D und/oder K, dem/den Hydrokolloid(en), Wasser und Zucker.

Das Hydrokolloid-Gel der erfindungsgemäßen Masse ist vorzugsweise im Wesentlichen frei von Emulgatoren. Dabei heißt "im Wesentlichen frei", dass, bezogen auf die Gesamtmasse an fettlöslichen Vitaminen, nicht mehr als 100 Masse-%, insbesondere nicht mehr als 50 Masse-%, z.B. nicht mehr als 20 Masse-% oder nicht mehr als 10 Masse-%, und im Optimalfall keinen Emulgator enthalten sind. Dadurch werden durch den Emulgator verursachte unerwünschte Nebenwirkungen auf das Verdauungssystem des Patienten (wie z.B. Durchfälle) verringert bzw. ganz vermieden.

Dabei bezeichnet der Begriff "Emulgatoren" grenzflächenaktive Substanzen, und insbesondere Polyoxyethylensorbitanfettsäureester, mit einem HLB-Wert (nach Griffin) im Bereich von 9-18 und v.a. im Bereich von 12-17, wie z.B. Polysorbat 20 (E432), Polysorbat 40 (E434), Polysorbat 60 (E435), Polysorbat 65 (E436) und Polysorbat 80 (E433).

Des Weiteren enthält die erfindungsgemäße Masse vorzugsweise keine Antioxidantien oder Konservierungsmittel, die ausgewählt sind unter den Klassen der Butylhydroxytoluole und Butylhydroxyanisole, Lecithin, Ethoxyquin, Methylparaben, Propylparaben, Sorbinsäure, Natriumbenzoat und Ascorbylpalmitat.

Die erfindungsgemäße Masse ist bevorzugt vegetarisch und/oder koscher und/oder halal und/oder allergenfrei und/oder glutenfrei und/oder frei Agentien, die BSE (Bovine spongiforme Enzephalopathie) und andere Formen von TSE (Transmissible Spongiforme Enzephalopathie) auslösen.

Entscheidend für die Bioverfügbarkeit der Vitamine ist das Auflöseverhalten der erfindungsgemäßen Masse. Es ist dafür vorteilhaft, dass ein beträchtlicher Anteil (bis zu 100%) der fettlöslichen Vitamine in der Masse in amorphem, gelöstem oder teilkristallinem Zustand vorliegt. Es hat sich überraschenderweise gezeigt, dass sich die fettlöslichen Vitamine und Provitamine in wässrigen Medien (wie z.B. 0,1 N Salzsäure) aus der erfindungsgemäßen Masse freisetzen lassen und dabei auch in Abwesenheit von Emulgatoren nicht spontan präzipitieren. Dadurch können die (Pro-)Vitamine vom Körper leicht aufgenommen werden.

Des Weiteren betrifft die vorliegende Erfindung die Verwendung der erfindungsgemäßen kaubaren und/oder lutschbaren Masse bei der Behandlung eines bestehenden oder drohenden Vitaminmangelzustands, wobei der Vitaminmangelzustand ein oder mehrere Vitamine betrifft, die in der Masse als Vitamine oder in Form entsprechender Provitamine enthalten sind.

Die erfindungsgemäße kaubare und/oder lutschbare Masse kann allgemein zur Behandlung von Patienten mit Maldigestion und/oder Malabsorption eingesetzt werden. Insbesondere kann die Masse zur Behandlung von Patienten mit altersbedingter funktioneller Malabsorption fettlöslicher Verbindungen, z.B. infolge einer gestörten Sekretion von Gallenflüssigkeit oder Enzymen der Bauchspeicheldrüse, sowie bei Patienten, die an Krankheiten und Zuständen leiden, welche den Dünndarm beeinträchtigen, Anwendung finden. Zu derartigen Krankheiten und Zuständen gehören unter anderem Morbus Crohn, Colitis ulcerosa, Mukoviszidose, Cholestasesyndrom, Zöliakie, Einheimische Sprue, Leberzirrhose, Alkoholkrankheit, Essstörungen, Dünndarmtumoren, Reizdarmsyndrom, Kurzdarmsyndrom und chronische entzündliche Darmerkrankungen. Des Weiteren kann die erfindungsgemäße Masse post-operativ nach Eingriffen am Gastrointestinaltrakt verwendet werden. Gemäß besonders bevorzugten Ausführungsformen betreffen die Verwendung der erfindungsgemäßen kaubaren und/oder lutschbaren Masse zur Behandlung eines bestehenden oder drohenden Vitaminmangels bei Menschen mit Mukoviszidose, Morbus Crohn, Colitis ulcerosa, Reizdarmsyndrom oder Alkoholkrankheit.

Die erfindungsgemäße Masse kann als Formkörper verabreicht werden, der vom Patienten entweder gelutscht oder zerbissen bzw. zerkaut und anschließend geschluckt wird. Dadurch ist die Masse speziell für Kinder und Patienten geeignet, die gewöhnliche Tabletten nicht schlucken können oder wollen. Da die Formkörper nicht im Ganzen geschluckt werden müssen, können sie ohne Wasser oder ein anderes Getränk verabreicht werden.

Ferner ist die Größe der Formkörper bedeutsam. Für eine einfache Verabreichung bei jungen Patienten (Kindern) sind die Formkörper aus erfindungsgemäßer Masse zweckmäßigerweise nicht länger als 2,5 cm (Länge), nicht breiter als 1,5 cm und nicht höher als 1,5 cm.

Bevorzugte Darreichungsformen der erfindungsgemäßen kaubaren und/oder lutschbaren Masse sind feste Bonbons, Kaugummis, Pastillen oder Drops und insbesondere kaubare Gummimassen (Kaubonbons, Fruchtgummis).

Die erfindungsgemäße kaubare und/oder lutschbare Masse kann durch ein einfaches Lösungsverfahren hergestellt werden, bei dem ein Gemisch, das ein oder mehrere Hydrokolloide, Wasser und ggf. weitere Bestandteile der Masse enthält, so lange erhitzt wird, bis sich das Hydrokolloid vollständig löst.

In seiner einfachsten Form umfasst das Verfahren zur Herstellung eines Fruchtgummis aus der erfindungsgemäßen Masse das Erhitzen eines Gemischs aus Hydrokolloid (z.B. Gelatine), Wasser und ggf. Süßungsmittel wie z.B. Glukose und Saccharose. Nach Erreichen einer Temperatur von weniger als 50°C werden die Vitamine und ggf. Aromen und weitere Bestandteile der Masse zugegeben und es wird gerührt, bis das Gemisch transluzent ist. Zur Formgebung kann die flüssige transluzente Masse dann z.B. in Puderkästen (mit Stärkepuder gefüllte Formen) gegossen werden. In der Regel wird die gegossene Masse danach einem Aushärtungs- und/oder Trocknungsprozess unterzogen. Die Wahl der Bedingungen für Luftfeuchtigkeit und Temperatur während der Aushärtung und/oder Trocknung kann dabei die Qualität der Fruchtgummis beeinflussen. Die Oberfläche von ausgehärteten Formkörpern aus erfindungsgemäßer Masse kann weiter behandelt werden. Z.B. können Fruchtgummis mit Bienenwachs beölt werden, um ihren typischen Glanz zu erhalten.

Drops aus erfindungsgemäßer Masse können beispielsweise mittels des Rotoformverfahrens hergestellt werden. Ferner kann die erfindungsgemäße Masse mittels Vertopfungsverfahren hergestellt werden, bei der eine Lösung der Bestandteile der Masse aus Düsen austritt, die mit Ultraschall in Vibration versetzt werden. Derartige Verfahren sind bekannt (siehe z.B. WO 2016/087276; und Glöckner (Hrsg.), Hobein und Lutz (Autoren), "Mikroverkapselung", Aulis Verlag, 1989).

Eine typische Zusammensetzung für die erfindungsgemäße Masse ist in Tabelle 1 angegeben.

**Tabelle 1.**

| Bestandteil | Menge in Masse-% |
|---|---|
| 10 mg β-Carotin, | 1,00 |
| 800,5 µg Retinylacetat | |
| 20 µg Vitamin D3, | |
| 100 mg Vitamin E¹⁾ | |
| 700 µg Vitamin K1 | |
| 75,7 mg Vitamin C als Antioxidans | |
| 10 mg Zn als Zinksulfat-Monohydrat | |
| Glukosesirup (Wassergehalt: 23 Masse-%) | 54,60 |
| Saccharose | 27,40 |
| Maltodextrin | 3,75 |
| Wasser | 3,00 |
| Schweinegelatine A²⁾ | 8,50 |
| Zitronensäure | 1,40 |
| Aroma (Orange) Beölungsmittel³⁾ | 0,20 0,15 |

| | |
|---|---|
| ¹⁾ D-α-Tocopherylacetat ²⁾ Schweinegelatine A: Gelstärke: 220 Bloom (6,67 Masse-% in Wasser, 10°C) Viskosität 3,5 mPa·s (6,67 Masse-% in Wasser, 50°C) Feuchtigkeit: 11,1 Masse-% (24 h, 550°C) Aschegehalt: 1 Masse-% (kolorimetrisch) Transparenz: 400 mm (5 Masse-% in Wasser, 40°C) pH-Wert: 5,5 (6,67 Masse-% in Wasser) ³⁾ Distelöl- & MCT-Öl-Pulver (Fettpulver auf Maltodextrinbasis mit etwa 33,3 Masse-% Distelöl und etwa 16,7 Masse-% MCT-Öl; MCT = mittelkettige Triglyceride aus Palm- und Kokosfett) | |

### Beispiel 1: Herstellung einer kaubaren Masse

Es wurden Formkörper einer kaubaren Masse mit der in Tabelle 1 angegebenen Zusammensetzung hergestellt. Dafür wurden zunächst 30 g Schweinegelatine A mit 50 g Wasser verrührt und bei Raumtemperatur 15 min quellen gelassen. Glukosesirup, Saccharose und Maltodextrin wurden gemischt und auf etwa 110°C erhitzt. Diese Zuckerlösung wurde unter Rühren nach und nach zu der gequollene Gelatinemasse gegeben, wobei darauf geachtet wurde, dass die Temperatur 50°C nicht überstieg. Anschließend wurde 30 min weitergerührt. Dann wurden die Vitaminbestandteile sowie das Zinksulfat zugegeben und bei einer Temperatur von 45-50°C weitergerührt. Nach Auflösung der festen Bestandteile wurde die Aromakomponente hinzugesetzt und die Masse in Formen ausgegossen. Innerhalb von etwa 2 h härteten die Formkörper aus und wurden anschließend bei etwa 30°C solange getrocknet bis der Wassergehalt auf einen Wert gesunken war, der einer Zusammensetzung gemäß Tabelle 1 entspricht. Nach Entnahme aus den Formen wurden der Formkörper mit Beölungsmittel behandelt. Die so erhaltenen Formkörper wogen etwa 2 g pro Formkörper.

### Beispiel 2: Physikalische Analyse der kaubaren Masse

Die in Beispiel 1 hergestellte kaubare Masse wurde 2 min in Flüssigstickstoff gekühlt und danach sofort in einer Schwingmühle (1 min, 30 Hz) zu Pulver zerschlagen. Das Pulver wurde nach dem Auftauen innerhalb von Minuten sehr klebrig. Das Pulver wurde mittels Durchlichtmikroskopie bei 100-facher Vergrößerung untersucht (Mikroskop Leica DMLM, Polfilter und Analysator zugeschaltet). Es konnten keinerlei kristalline Bestandteile im Pulver beobachtet werden.

Pulver der gemäß Beispiel 1 hergestellten kaubaren Masse wurde in 0,1 M Salzsäure suspendiert (1 g Pulver pro 100 ml 0,1 M Salzsäure) und drei Stunden gerührt. Das Pulver löste sich vollständig auf.

### Beispiel 3: Chemische Analyse der kaubaren Masse

Der β-Carotingehalt von Formkörpern der in Beispiel 1 hergestellten kaubare Masse wurde mittels HPLC/UV bestimmt (Methode: DIN EN 12823-2). Es wurde eine Doppelbestimmung durchgeführt, die einen Mittelwert von 0,95 mg β-Carotin pro Formkörper ergab (theoretischer Wert: 1 mg β-Carotin pro Formkörper). β-Carotin wurde als Leitvitamin zur Gehalts- und Stabilitätsbestimmung gewählt, da es lichtempfindlich ist und oxidativ abgebaut wird. Nach einmonatiger Lagerung der kaubaren Masse bei Kühlung auf etwa 4°C im Dunklen wurde kein Abbau von β-Carotin festgestellt.

### Beispiel 4: Geschmackstest

Formkörper der erfindungsgemäßen kaubaren Masse gemäß Beispiel 1 in Gestalt von Gummibären **(B2)** wurden in Vergleich zu einer kommerziell erfolgreichen Süßware, ebenfalls in Gestalt bärenförmiger Fruchtgummis, (Haribo Goldbären, **B1)** von einer Gruppe bestehend aus 10 Personen im Alter von 6-25 Jahren getestet. Den Testpersonen wurde mitgeteilt, dass es sich bei den Proben um vitaminhaltige Gummibären handelt. Die Verkostung erfolgte blind, d.h. ohne dass die Probanden erkennen konnten, welche Bären sie zu sich nahmen.

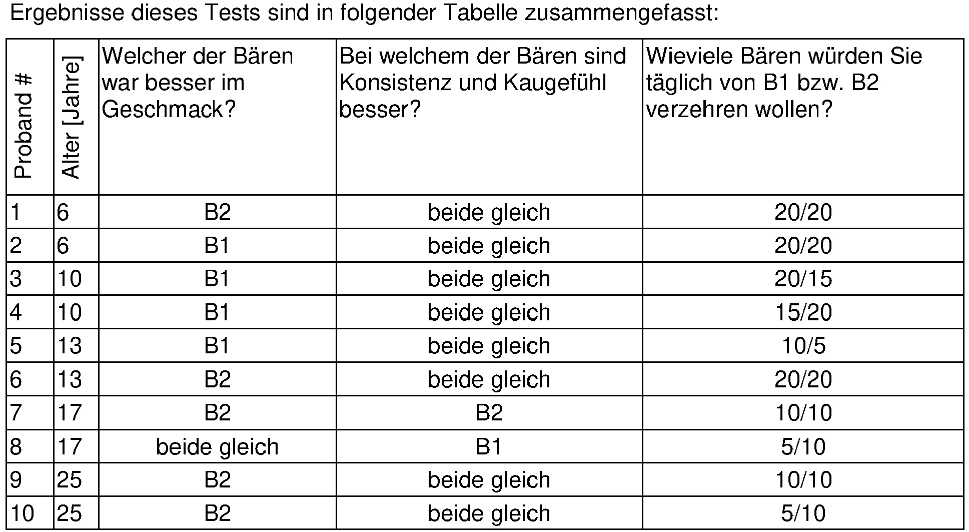

### Beispiel 5: Freisetzungsuntersuchung

Pro Versuch wurden jeweils 10 Formkörper der erfindungsgemäßen kaubaren Masse gemäß Beispiel 1 in Gestalt von Gummibären in 1000 ml 0,1 M wässriger Salzsäure bei 36,5±0,5°C gegeben und bei 100 Upm gerührt. Dabei wurde 0, 1, 2 und 4 Stunden nach Versuchsstart die Menge an in Lösung befindlichem Vitamin-A-Acetat mittels HPLC und UV-Detektion bestimmt. Dazu wurden jeweils 10 ml Probelösung entnommen und das entnommene Volumen mit einer entsprechenden Menge 0,1 M wässriger Salzsäure ersetzt, um das Gesamtvolumen des Versuchsansatzes konstant zu halten. Die Ergebnisse sind in folgender Tabelle zusammengefasst:

| Zeitpunkt der Probennahme [Stunden nach Versuchsstart] | in Lösung befindliches Vitamin-A-Acetat [mg/l] | | | | Freisetzung [%] |
|---|---|---|---|---|---|
| | Versuch 1 | Versuch 2 | Versuch 3 | Mittelwert | |
| 0 | 0,000 | 0,000 | 0,000 | 0,000 | 0,0 |
| 1 | 0,784 | 0,752 | 0,759 | 0,765 | 104,8 |
| 2 | 0,800 | 0,812 | 0,818 | 0,810 | 111,0 |
| 4 | 0,781 | 0,805 | 0,779 | 0,788 | 108,0 |

## Patentansprüche

1. Kaubare und/oder lutschbare Masse auf Basis eines Hydrokolloid-Gels, enthaltend
- Retinylacetat und β-Carotin,
- mindestens ein Vitamin oder Provitamin ausgewählt unter den Vitaminen D, E und K sowie Provitaminen dieser Vitamine,
- Zink,
wobei
- die Gesamtmenge an den Vitaminen A, D, E und K und den Provitaminen dieser Vitamine mindestens 0,5 Masse-% der Gesamtmasse des Hydrokolloid-Gels beträgt,
- die Gesamtmenge an Vitamin A und Provitamin A mindestens 0,05 Masse-% der Gesamtmasse des Hydrokolloid-Gels beträgt,
- die Masse 0,00-0,6 Masse-%, bezogen auf die Gesamtmasse des Hydrokolloid-Gels, Vitamin C enthält,
- der Wassergehalt des Hydrokolloid-Gels 1-35 Masse-% beträgt, und
- die Vitamine und Provitamine im Hydrokolloid-Gel in nicht Emulgator-solubilisierter Form vorliegen.

2. Masse nach Anspruch 1, enthaltend mindestens ein Vitamin D oder Provitamin D.

3. Masse nach Anspruch 1, enthaltend mindestens ein Vitamin E oder Provitamin E, mindestens ein Vitamin D oder Provitamin D, und mindestens ein Vitamin K oder Provitamin K.

4. Masse nach Anspruch 2 oder 3, wobei die Gesamtmenge an Vitamin D oder Provitamin D mindestens 0,0003 Masse-% der Gesamtmasse des Hydrokolloid-Gels beträgt.

5. Masse nach einem der Ansprüche 1-4, wobei das Hydrokolloid ausgewählt ist unter Pektinen, Gummiarabikum, Galactomannanen, Agar, Carrageen, Alginaten, Alginsäuren, Gelatine, Xanthan, Traganth und Gemischen davon.

6. Masse nach Anspruch 5, wobei das Hydrokolloid ausgewählt ist unter Pektinen und Gelatine.

7. Masse nach einem der Ansprüche 1-6, wobei die Menge an Zink im Bereich von 0,001 - 0,1 Masse-% bezogen auf die Gesamtmasse des Hydrokolloid-Gels liegt.

8. Masse nach einem der Ansprüche 1-7, enthaltend 0,01-0,5 Masse-% Vitamin C bezogen auf die Gesamtmasse des Hydrokolloid-Gels.

9. Masse nach einem der Ansprüche 1-8, wobei mindestens 50 Masse-% der Gesamtmenge an den Vitaminen und Provitaminen in nicht-kristalliner Form vorliegen.

10. Masse nach einem der Ansprüche 1-9 zur Verwendung bei der Behandlung eines bestehenden oder drohenden Vitaminmangelzustands betreffend ein oder mehrere Vitamine, die der Masse als Vitamine oder in Form entsprechender Provitamine enthalten sind.

11. Masse zur Verwendung nach Anspruch 10, wobei die Masse bei der Behandlung eines bestehenden oder drohenden Vitaminmangelzustands bei einem Menschen mit Mukoviszidose, Morbus Crohn, Colitis ulcerosa, Reizdarmsyndrom oder Alkoholkrankheit eingesetzt wird.

## Claims

1. Chewable and/or suckable mass based on a hydrocolloid gel, containing
- retinyl acetate and β-carotene,
- at least one vitamin or provitamin selected from the vitamins D, E and K and provitamins of these vitamins,
- zinc,
wherein
- the total amount of the vitamins A, D, E and K and the provitamins of these vitamins is at least 0.5% by mass of the total mass of the hydrocolloid gel,
- the total amount of vitamin A and provitamin A is at least 0.05% by mass of the total mass of the hydrocolloid gel,
- based on the total mass of the hydrocolloid gel, the mass contains 0.00-0.6% by mass of vitamin C,
- the water content of the hydrocolloid gel is 1-35% by mass, and
- the vitamins and provitamins are present in the hydrocolloid gel in a form that is not emulsifier-solubilised.

2. Mass according to claim 1, containing at least one vitamin D or provitamin D.

3. Mass according to claim 1, containing at least one vitamin E or provitamin E, at least one vitamin D or provitamin D, and at least one vitamin K or provitamin K.

4. Mass according to claim 2 or 3, wherein the total amount of vitamin D or provitamin D is at least 0.0003% by mass of the total mass of the hydrocolloid gel.

5. Mass according to one of claims 1-4, wherein the hydrocolloid is selected from pectins, gum arabic, galactomannans, agar, carrageenan, alginates, alginic acids, gelatines, xanthan, tragacanth and mixtures thereof.

6. Mass according to claim 5, wherein the hydrocolloid is selected from pectins and gelatines.

7. Mass according to one of claims 1-6, wherein the amount of zinc ranges from 0.001-0.1% by mass based on the total mass of the hydrocolloid gel.

8. Mass according to one of claims 1-7, containing 0.01-0.5% by mass of vitamin C based on the total mass of the hydrocolloid gel.

9. Mass according to one of claims 1-8, wherein at least 50% by mass of the total mass of the vitamins and provitamins is present in non-crystalline form.

10. Mass according to one of claims 1-9 for use when treating an existing or imminent state of vitamin deficiency relating to one or more vitamins that are contained in the mass as vitamins or in the form of corresponding provitamins.

11. Mass for use according to claim 10, wherein the mass is used when treating an existing or imminent state of vitamin deficiency in a human with cystic fibrosis, Crohn's disease, ulcerative colitis, irritable bowel syndrome or alcoholism.

## Revendications

1. Pâte à base de gel hydrocolloïde susceptible d'être mâchée et/ou sucée, contenant :
- de l'acétate de rétinyle et de la β-carotène,
- au moins une vitamine ou provitamine choisie parmi les vitamines D, E et K ainsi que les provitamines de ces vitamines,
- du zinc,
étant entendu que :
- la quantité totale de vitamines A, D, E et K et de provitamines de ces vitamines représente au moins 0,5 % en masse de la masse totale de gel hydrocolloïde,
- la quantité totale de vitamine A et de provitamine A représente au moins 0,05 % en masse de la masse totale de gel hydrocolloïde,
- la pâte contient 0,00 à 0,6 % en masse de vitamine C, par rapport à la masse totale de gel hydrocolloïde,
- la teneur en eau du gel hydrocolloïde représente 1 à 35 % en masse, et
- les vitamines et provitamines sont présentes dans le gel hydrocolloïde sous une forme non solubilisée par émulsifiant.

2. Pâte selon la revendication 1, contenant au moins une vitamine D ou provitamine D.

3. Pâte selon la revendication 1, contenant au moins une vitamine E ou provitamine E, au moins une vitamine D ou provitamine D, et au moins une vitamine K ou provitamine K.

4. Pâte selon la revendication 2 ou 3, dans laquelle la quantité totale de vitamine D ou de provitamine D représente au moins 0,0003 % en masse de la masse totale de gel hydrocolloïde.

5. Pâte selon l'une des revendications 1 à 4, dans laquelle l'hydrocolloïde est choisi parmi les pectines, la gomme arabique, les galactomannanes, l'agar-agar, les carraghénanes, les alginates, les acides alginiques, la gélatine, la gomme xanthane, la gomme adragante et leurs mélanges.

6. Pâte selon la revendication 5, dans laquelle l'hydrocolloïde est choisi parmi les pectines et la gélatine.

7. Pâte selon l'une des revendications 1 à 6, dans laquelle la quantité de zinc se situe dans la plage de 0,001 à 0,1 % en masse par rapport à la masse totale de gel hydrocolloïde.

8. Pâte selon l'une des revendications 1 à 7, contenant 0,01 à 0,5 % en masse de vitamine C par rapport à la masse totale de gel hydrocolloïde.

9. Pâte selon l'une des revendications 1 à 8, dans laquelle au moins 50 % en masse de la quantité totale de vitamines et provitamines se trouve sous forme non cristalline.

10. Pâte selon l'une des revendications 1 à 9 destinée à être utilisée dans le traitement d'un état de carence vitaminique existant ou en prévention, concernant une ou plusieurs vitamines contenues dans la pâte en tant que vitamines ou sous la forme de provitamines correspondantes.

11. Pâte destinée à être utilisée selon la revendication 10, ladite pâte étant utilisée dans le traitement d'un état de carence vitaminique existant ou en prévention chez l'homme atteint de mucoviscidose, maladie de Crohn, colite ulcéreuse, syndrome du côlon irritable ou alcoolisme.
